# EUROPÄISCHE PATENTSCHRIFT

(11) **EP 0 424 764 B1**
(45) Veröffentlichungstag und Bekanntmachung des Hinweises auf die Patenterteilung: **26.01.1994**
(21) Anmeldenummer: 90119661.8
(22) Anmeldetag: 13.10.1990
(51) Int. Cl.: C07C 209/48, C07C 211/14

(54) **Verfahren zur Herstellung von Bis- und Tris-(3-dimethylaminopropyl)-amin**
Process for the preparation of Bis- and Tris-(3-dimethylaminopropyl)amine
Procédé pour la préparation de Bis- et Tris-(3-dimethylaminopropyle)amine

(30) Priorität: 26.10.1989 DE 3935641
(43) Veröffentlichungstag der Anmeldung: 02.05.1991
(73) Patentinhaber: BAYER AG, 51368 Leverkusen (DE)
(72) Erfinder: Käsbauer, Josef, Dr., W-5632 Wermelskirchen 2 (DE); Fiege, Helmut, Dr., W-5090 Leverkusen (DE); Kiel, Wolfgang, Dr., W-5068 Odenthal (DE)

(56) Entgegenhaltungen:
- DE-A- 3 048 832

## Beschreibung

Die vorliegende Erfindung betrifft ein Verfahren zur Herstellung von Bis- und Tris-(3-dimethylaminopropyl)-amin durch katalytische Hydrierung von 3-(Dimethylamino)-propionitril. Hierbei werden Palladium-Katalysatoren auf einem Al₂O₃-haltigen Träger eingesetzt.

Bis- und Tris-(3-dimethylaminopropyl)-amine sind wichtige Hilfsstoffe zur Herstellung von Polyurethanschaumstoffen (DE-OS 30 48 832, DE-OS 26 24 528, US-4 255 528, US-4 431 753). Die Herstellung von Bis-(3-dimethylaminopropyl)-amin wird beispielsweise in US-4 101 466, US-4 143 071 und DE-OS 30 48 832 beschrieben. In allen Fällen werden jedoch nur niedrige Produktausbeuten erhalten oder die Selektivität ist nur bei geringem Umsatz zufriedenstellend. Die Produktreinigung ist folglich aufwendig und für ein technisches Verfahren nicht geeignet.

Die Herstellung von Tris-(3-dimethylaminopropyl)-amin wird beispielsweise in DE-PS 26 24 528 beschrieben. Dabei wird N,N-Bis-(3-dimethylaminopropyl)-propylendiamin-1,3 nach Leuckart-Wallach mit Formaldehyd in Gegenwart von Ameisensäure methyliert. Diese Herstellungsmethode geht jedoch von einem teureren Zwischenprodukt aus und benötigt korrosive und aggressive Chemikalien.

Nach J, Chem. Soc. (A) 1971, 2024 wird das im vorigen Absatz erwähnte tertiäre Amin durch Umsetzung von Tris-(3-hydroxypropyl)-amin mit Thionylchlorid und anschließend mit Dimethylamin-Überschuß hergestellt. Die Reaktionsdauer ist mit 24 Stunden sehr lang. Zur Aufarbeitung wird die Reaktionslösung mit überschüssiger 40 %iger Kaliumhydroxid-Lösung versetzt und mit Ether extrahiert. Diese Herstellungsmethode ist vom technischen und ökologischen Standpunkt her nicht praktikabel. Es werden auch keine Ausbeuten genannt.

Somit ist also kein Reaktionsweg zur Herstellung der im Titel genannten Stoffe beschrieben, der von einfachen und preisgünstigen Verbindungen ausgeht und das tertiäre Amin in guten Ausbeuten liefert. Somit bestand ein Bedürfnis nach einem solchen Verfahren.

Es wurde ein Verfahren zur Herstellung von Bis-(3-dimethylaminopropyl)-amin und Tris-(3-dimethylaminopropyl)-amin durch katalytische Hydrierung von 3-(Dimethylamino)-propionitril gefunden, das dadurch gekennzeichnet ist, daß man einen Palladium-Katalysator auf einem Al₂O₃-haltigen Träger einsetzt.

Als Al₂O₃-haltige Träger sei beispielsweise γ-Al₂O₃ oder Spinell genannt. Der Palladium-Gehalt eines solchen Katalysators liegt bei 0,1-10 Gew.-%, bevorzugt 0,5-5 Gew.-%, bezogen auf das Gesamtgewicht des Katalysators.

Die Temperatur für das erfindungsgemäße Verfahren beträgt 50-150°C. Der Druck im erfindungsgemäßen Verfahren beträgt 50-300 bar.

Im erfindungsgemäßen Verfahren wird ein Gemisch aus dem genannten Bis-amin und dem genannten Tris-amin erhalten, die in bekannter Weise, beispielsweise destillativ, getrennt werden können.

Die Vorteile des erfindungsgemäßen Verfahrens liegen in der hohen Ausbeute an den genannten Verbindungen, in der Durchführung als einstufiges Verfahren (Eintopfverfahren), im leicht zugänglichen Katalysator, der in einer dem Fachmann grundsätzlich bekannten Weise, beispielsweise durch Tränkung des Trägers mit Palladium-Verbindungen, hergestellt werden kann, in der langen Standzeit des Katalysators bei unveränderter Aktivität und in der einfachen Aufarbeitung des Reaktionsgemisches.

Die Produktausbeuten steigen mit erhöhtem Druck und mit gleichzeitig oder unabhängig davon abgesenkter Temperatur.

Im Rahmen des erfindungsgemäßen Verfahrens wurden weiterhin einige interessante Varianten gefunden, die es gestatten, bei wechselndem Bedarf an dem Bis-amin oder dem Tris-amin das jeweils gewünschte in höherer Ausbeute zu erhalten. So wurde gefunden, daß bei Verwendung von Al₂O₃ als Katalysatorträger sich bevorzugt das sekundäre Bis-amin bildet, während ein Spinell als Trägermaterial verstärkt zum tertiären Tris-amin führt.

Wenn zur Erhöhung der Selektivität für das Bis-amin Al₂O₃ als Katalysatorträger eingesetzt wird, ist ein solches mit großer Oberfläche, beispielsweise 200-500 m²/g, bevorzugt 300-450 m²/g, vorteilhaft, weil bei kleineren inneren Oberflächen die Aktivität eines solchen Katalysators rascher abnimmt.

Bei Verwendung eines Spinells als Träger für den Palladium-Katalysator wird das genannte Tris-amin als Hauptprodukt erhalten. Als spinellbildende Elemente kommen beispielsweise Li, Be, Mg, Zn, Mn, Cu und Ni in Betracht. In bevorzugter Weise sei LiAl-Spinell als Trägermaterial genannt.

In einer weiteren wichtigen Variante des erfindungsgemäßen Verfahrens kann die Einsatzmischung für das erfindungsgemäße Verfahren zur Beeinflussung der Produktzusammensetzung benutzt werden. Hierzu dient 3-Dimethylamino-propylamin, das sich durch alleinige Hydrierung der Nitrilgruppe des 3-(Dimethylamino)-propionitrils bildet. So führt eine Mischung im etwa stöchiometrischen Bereich aus 3-(Dimethylamino)- propionitril und 3-Dimethylamino-propylamin zur verstärkten Bildung des sekundären Bis-amins, während ein Überschuß an Nitril oder der alleinige Einsatz von Nitril das tertiäre Tris-amin als Hauptprodukt liefert.

Selbstverständlich können die genannten Varianten (verschiedene Trägermaterialien und/oder Mitverwendung von 3-Dimethylamino-propylamin oder dessen Auslassung) miteinander kombiniert werden, wobei Verstärkungen in der gewünschten Richtung erzielt werden.

Bei der Mitverwendung von 3-Dimethylamino-propylamin wird dieses in einer Menge von 20-80 Mol-%, bevorzugt 40-60 Mol-%, bezogen auf das Gemisch von 3-Dimethylamino-propylamin und 3-(Dimethylamino)-propionitril, eingesetzt.

### Beispiel 1

In einem 0,3 l VA-Autoklav wurden 0,5 Mol 3-(Dimethylamino)-propionitril und 0,5 Mol 3-(Dimethylamino)-propylamin unter Zusatz von 3 g eines Katalysators bestehend aus 3,7 % Pd auf γ-Al₂O₃ (Oberfläche 400 m²) bei 80-90°C und 100 bar hydriert. Nach 2 Stunden Hydrierzeit erhielt man 93 g eines Produktgemisches, das sich aus 81,5 % Bis-(3-dimethylaminopropyl)-amin und 3,8 % Tris-(3-dimethylaminopropyl)-amin zusammensetzte. Die beiden Produkte konnten leicht destillativ getrennt werden. Der Katalysator konnte wiederholt eingesetzt werden.

### Beispiel 2

In einem 0,7 l VA-Autoklav wurde 1 Mol 3-(Dimethylamino)-propionitril unter Zusatz von 3 g des Katalysators aus Beispiel 1 bei 120°C und 100 bar hydriert. Nach 2,5 Stunden Hydrierzeit erhielt man 88 g Produktgemisch, das sich aus 32 % sekundärem und 48 % tertiärem Amin zusammensetzte.

### Beispiel 3

In einem 3 l VA-Autoklav wurden 10 Mol 3-(Dimethylamino)-propionitril und 30 g Katalysator, bestehend aus 4 % Pd auf LiAl-Spinell bei 120°C und 220 bar hydriert. Nach 1,5 Stunden Hydrierzeit wurden 860 g Produktgemisch isoliert, das 31 % sekundäres und 58 % tertiäres Amin enthielt.

### Beispiel 4 (Vergleichsbeispiel)

Wie in Beispiel 1 wurden 0,1 Mol 3-(Dimethylamino)-propionitril und 0,9 Mol 3-Dimethylaminopropylamin hydriert. Nach 2 Stunden Hydrierzeit erhielt man 95,5 g eines Produktgemisches, das sich nur aus 21,5 % sekundärem und 0,1 % tertiärem Amin zusammensetzte.

## Patentansprüche

1. Verfahren zur Herstellung von Bis-(3-dimethylaminopropyl)-amin und Tris-(3-dimethylaminopropyl)-amin, dadurch gekennzeichnet, daß man 3-(Dimethylamino)-propionitril katalytisch an einem Palladium-Katalysator auf einem Al₂O₃-haltigen Träger hydriert.

2. Verfahren nach Anspruch 1, dadurch gekennzeichnet, daß als Al₂O₃-haltige Träger γ-Al₂O₃ oder ein Spinell eingesetzt werden.

3. Verfahren nach Anspruch 1, dadurch gekennzeichnet, daß der Palladium-Gehalt 0,1-10 Gew.-%, bevorzugt 0,5-5 Gew.-%, bezogen auf das Gesamtgewicht des Katalysators, beträgt.

4. Verfahren nach Anspruch 1, dadurch gekennzeichnet, daß man zur Erhöhung der Selektivität für Bis-(3-dimethylaminopropyl)-amin als Träger Al₂O₃ einsetzt.

5. Verfahren nach Anspruch 4, dadurch gekennzeichnet, daß man ein Al₂O₃ mit einer Oberfläche von 200-500 m²/g, bevorzugt 300-450 m²/g, einsetzt.

6. Verfahren nach Anspruch 1, dadurch gekennzeichnet, daß man zur Erhöhung der Selektivität an Tris-(3-dimethylaminopropyl)-amin als Träger einen Spinell einsetzt.

7. Verfahren nach Anspruch 6, dadurch gekennzeichnet, daß als Träger LiAl-Spinell eingesetzt wird.

8. Verfahren nach Anspruch 1, dadurch gekennzeichnet, daß zur stärkeren Bildung von Bis-(3-dimethylaminopropyl)-amin das 3-(Dimethylamino)-propionitril im Gemisch mit 3-Dimethylamino-propylamin eingesetzt wird, wobei 3-Dimethylamino-propylamin 20-80 Mol-% dieses Gemisches ausmacht.

9. Verfahren nach Anspruch 8, dadurch gekennzeichnet, daß 40-60 Mol-% 3-Dimethylamino-propylamin eingesetzt werden.

## Claims

1. Process for the preparation of bis-(3-dimethylaminopropyl)-amine and tris-(3-dimethylaminopropyl)-amine, characterized in that 3-(dimethylamino)-propionitrile is catalytically hydrogenated with a palladium catalyst on an Al₂O₃-containing support.

2. Process according to Claim 1, characterized in that γ-Al₂O₃ or a spinel is employed as the Al₂O₃-containing support.

3. Process according to Claim 1, characterized in that the palladium content is 0.1-10 % by weight, preferably 0.5-5 % by weight, relative to the total weight of the catalyst.

4. Process according to Claim 1, characterized in that Al₂O₃ is employed as the support to increase the selectivity for bis-(3-dimethylaminopropyl)-amine.

5. Process according to Claim 4, characterized in that an Al₂O₃ having a surface area of 200-500 m²/g, preferably 300-450 m²/g, is employed.

6. Process according to Claim 1, characterized in that a spinel is employed as the support to increase the selectivity for tris-(3-dimethylaminopropyl)-amine.

7. Process according to Claim 6, characterized in that LiAl spinel is employed as the support.

8. Process according to Claim 1, characterized in that the 3-(dimethylamino)-propionitrile is employed in a mixture with 3-dimethylamino-propylamine for greater formation of bis-(3-dimethylaminopropyl)-amine, 3-dimethylamino-propylamine making up 20-80 mol-% of this mixture.

9. Process according to Claim 8, characterized in that 40-60 mol-% of 3-dimethylamino-propylamine is employed.

## Revendications

1. Procédé de préparation de bis-(3-diméthylaminopropyl)-amine et de tris-(3-diméthylaminopropyl)-amine, caractérisé en ce que l'on effectue l'hydrogénation catalytique du 3-diméthylamino -propionitrile par un catalyseur au palladium sur un support à base de Al₂O₃.

2. Procédé selon la revendication 1,caractérisé en ce que l'on utilise comme support à base de Al₂O₃ du Al₂O₃ ou un spinelle.

3. Procédé selon la revendication 1,caractérisé en ce que la teneur en palladium est comprise entre 0,1 et 10% en poids et, de préférence, entre 0,5 et 5% en poids rapportés au poids total du catalyseur.

4. Procédé selon la revendication 1,caractérisé en ce que l'on utilise Al₂O₃ comme support afin d'augmenter la sélectivité pour la bis-(3-diméthylaminopropyl)-amine.

5. Procédé selon la revendication 4,caractérisé en ce que l'on utilise un Al₂O₃ ayant une surface de 200 à 500 m²/g et, de préférence, de 300 à 450 m²/g.

6. Procédé selon la revendication 1,caractérisé en ce que l'on utilise un spinelle comme support pour augmenter la sélectivité pour la tris-(3-diméthylaminopropyl)-amine.

7. Procédé selon la revendication 6,caractérisé en ce que l'on utilise un spinelle de LiAl comme support.

8. Procédé selon la revendication 1,caractérisé en ce que,pour augmenter la formation de bis-(3-diméthylaminopropyl)-amine, on utilise le 3- diméthylamino - propionitrile en mélange avec la 3-diméthylaminopropyl - amine, la 3-diméthylaminopropyl -amine constituant de 20 a 80 moles-% de ce mélange.

9. Procédé selon la revendication 8,caractérisé en ce que l'on utilise de 40 à 60 moles-% de 3-diméthylaminopropyl -amine.
